(19) Europäisches Patentamt
European Patent Office
Office européen des brevets

(11) **EP 4 555 995 B1**

(12) **FASCICULE DE BREVET EUROPEEN**

(45) Date de publication et mention de la délivrance du brevet:
**10.06.2026 Bulletin 2026/24**

(21) Numéro de dépôt: **23315419.4**

(22) Date de dépôt: **14.11.2023**

(51) Classification Internationale des Brevets (IPC):
***A61K 8/36*** *(2006.01)* ***A61K 8/9778*** *(2017.01)*
***A61K 31/201*** *(2006.01)* ***A61K 45/06*** *(2006.01)*
***A61P 17/00*** *(2006.01)* ***A61P 17/02*** *(2006.01)*
***A61Q 5/08*** *(2006.01)* ***A61Q 19/02*** *(2006.01)*
***A61K 36/17*** *(2006.01)* ***A61K 36/185*** *(2006.01)*
***A61K 36/28*** *(2006.01)* ***A61K 36/31*** *(2006.01)*
***A61K 36/45*** *(2006.01)* ***A61K 36/484*** *(2006.01)*
***A61K 36/60*** *(2006.01)* ***A61K 36/87*** *(2006.01)*

(52) Classification Coopérative des Brevets (CPC):
(C-Sets disponibles)
**A61Q 19/02; A61K 8/361; A61K 8/9778; A61K 31/201; A61K 36/185; A61K 36/28; A61K 36/31; A61K 36/45; A61K 36/484; A61K 36/60; A61K 36/87; A61K 45/06; A61P 17/00; A61P 17/02; A61Q 5/08** (Cont.)

(54) **UTILISATION COSMÉTIQUE D'UN ACIDE GRAS CYCLOPROPÉNIQUE POUR INHIBER L'ACTIVITÉ DE LA SCD**

KOSMETISCHE VERWENDUNG EINER CYCLOPROPENFETTSÄURE ZUR HEMMUNG DER SCD-AKTIVITÄT

COSMETIC USE OF A CYCLOPROPENE FATTY ACID FOR INHIBITING SCD ACTIVITY

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(43) Date de publication de la demande:
**21.05.2025 Bulletin 2025/21**

(73) Titulaire: **Laboratoires d'Anjou**
**75008 Paris (FR)**

(72) Inventeur: **Foussé, Carole**
**75008 PARIS (FR)**

(74) Mandataire: **Yes My Patent**
**32 Boulevard Richard Lenoir**
**75011 Paris (FR)**

(56) Documents cités:
**WO-A1-2009/067095**
**WO-A1-2011/163560**
**WO-A1-2018/142033**
**WO-A2-2009/106991**

- **PELÁEZ RAFAEL ET AL: "Sterculic Acid: The Mechanisms of Action beyond Stearoyl-CoA Desaturase Inhibition and Therapeutic Opportunities in Human Diseases", vol. 9, no. 1, 7 January 2020 (2020-01-07), pages 140, XP093146973, ISSN: 2073-4409, Retrieved from the Internet <URL:https://www.semanticscholar.org/reader/c67e5b66ba20b1491ec5d6daa07c1bb834cffb06> DOI: 10.3390/cells9010140**
- **AYUNINGTYAS I N ET AL: "The study of safety and skin whitening efficacy of melinjo (Gnetum gnemon L.) seed extract-loaded lipid particle gel", PHARMACOGNOSY RESEARCH, PHARMACOGNOSY NETWORK WORLDWIDE, BANGALORE, IN, 1 May 2018 (2018-05-01), pages 432 - 436, XP018531284, ISSN: 0974-8490**

EP 4 555 995 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC): (Cont.)

C-Sets
**A61K 36/185, A61K 2300/00;**
**A61K 36/28, A61K 2300/00;**
**A61K 36/31, A61K 2300/00;**
**A61K 36/45, A61K 2300/00;**
**A61K 36/484, A61K 2300/00;**
**A61K 36/60, A61K 2300/00;**
**A61K 36/87, A61K 2300/00**

## Description

L'invention concerne l'utilisation cosmétique non-thérapeutique d'une composition comprenant au moins un acide gras cyclopropénique pour inhiber l'activité de la stéaroyl-CoA désaturase, et agir comme agent dépigmentant, éclaircissant et/ou blanchissant de la peau, des muqueuses, des poils et/ou des cheveux humains et/ou comme agent permettant d'éliminer les taches pigmentaires de la peau humaine. L'invention concerne également l'utilisation dermatologique non-thérapeutique de cette composition dans le traitement des hypermélanoses. Selon un mode de réalisation, ledit au moins un acide gras cyclopropénique est choisi parmi l'acide sterculique et/ou l'acide malvalique.

L'invention est du domaine de la cosmétique et/ou de la dermatologie. Elle trouve une application particulièrement avantageuse dans le domaine des soins de la peau, y compris la dépigmentation ou le blanchiment de la peau.

## Art Antérieur

La pigmentation de la peau est fonction des saisons de l'année, des groupes ethniques et du sexe, mais surtout de multiples facteurs telle que l'hémoglobine des vaisseaux, les caroténoïdes du derme et principalement de la concentration de mélanine produite par les mélanocytes de la couche basale, sous l'action de la tyrosinase, du cuivre et de l'oxygène.

Le mécanisme de formation de la pigmentation de la peau est particulièrement complexe et fait intervenir schématiquement les principales étapes suivantes :

Tyrosine ---> Dopa ---> Dopaquinone ---> Dopachrome ---> Mélanine

La tyrosinase est l'enzyme essentielle intervenant dans cette suite de réactions.

Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Deux types chimiques de mélanines sont produits :

- Les eumélanines, qui sont des polymères d'indole-5-6-quinone, de couleur brune ou noire ;
- Les phéomélanines, qui sont des composés contenant près de 10% de soufre et de structure polymère de la cystéinyl dopa, de couleur jaune-rouge.

La couleur de la peau d'un individu est notamment fonction de ce rapport eu-mélanine/phéomélanine.

Sous l'effet de stimulations exogènes ou endogènes, il peut apparaître des modifications de la teinte de la peau, appelées hyperchromie et hypochromie, en fonction d'une hyper ou d'une hypopigmentation.

Les hyperchromies sont des accumulations de pigments mélaniques, de caroténoïdes ou de pigments exogènes, pouvant être liées à des hypermélanoses telles que le mélasma ou les lentigos dus au vieillissement cutané et/ou à l'exposition solaire.

Les motivations qui poussent à décolorer la peau peuvent être différentes, et revêtir davantage un aspect cosmétique. L'éclaircissement franc du teint est notamment recherché en Afrique Noire, et la pâleur ou la blancheur du visage en Asie.

Ainsi depuis un bon nombre d'années, différentes molécules ont déjà été utilisées et testées comme agent éclaircissant, dépigmentant ou blanchissant.

Les agents éclaircissants, dépigmentant ou blanchissant de la peau, des poils et/ou des cheveux humains sont des composés capables d'agir à l'échelon tissulaire, cellulaire ou subcellulaire. Les substances les plus utilisées en tant que dépigmentant dans des compositions sont notamment des composés tels que la vitamine C, les dérivés de vitamine C, l'arbutine, l'hydroquinone, ou l'acide kojique.

Ces différents composés sont connus pour agir sur la synthèse et/ou l'activité de la tyrosinase, ou pour réduire la quantité de mélanine formée ou encore pour stimuler l'élimination de la mélanine au travers des kératinocytes. Malheureusement, ils sont soit toxiques, cas de l'hydroquinone, soit instables en solution.

Ayuningtyas et al. divulgent dans Pharmacognosy Research, 2018, 432-436 que l'extrait de graines de Melinjo (Gnetum gnemon L.) est potentiellement un agent blanchissant pour la peau.

WO-A-2018142033 décrit des compositions dermatologiques ou cosmétiques à action dépigmentante par application topique comprenant l'association de six constituants.

Ainsi, il subsiste le besoin d'un nouvel agent éclaircissant, dépigmentant ou blanchissant de la peau, des poils et/ou des cheveux humains, ayant une action au moins aussi efficace que ceux connus, mais n'ayant pas leurs inconvénients.

D'une manière surprenante, la Demanderesse a mis en évidence un nouveau mécanisme d'action permettant une action dépigmentante. En particulier, l'utilisation d'acides gras cyclopropéniques est capable d'inhiber l'activité de la stéaroyl-CoA désaturase, et ainsi avoir une action éclaircissante, dépigmentante et/ou blanchissante, de la peau, des

muqueuses, des poils et/ou des cheveux humains, et une action d'élimination des taches pigmentaires sur la peau humaine.

L'utilisation de tels acides gras cyclopropéniques pour d'inhiber l'activité de la stéaroyl-CoA afin d'obtenir un effet éclaircissant, dépigmentant, blanchissant et/ou antitache représente le concept général unique de cette invention.

## Exposé de l'invention

Ainsi, selon un premier aspect, l'invention concerne l'utilisation cosmétique non-thérapeutique d'une composition comprenant au moins un acide gras cyclopropénique pour inhiber l'activité de la stéaroyl-CoA désaturase.

Selon l'invention, ledit acide gras cyclopropénique agit comme agent dépigmentant, éclaircissant et/ou blanchissant de la peau, des muqueuses, des poils et/ou des cheveux humains et/ou comme agent permettant d'éliminer les taches pigmentaires de la peau humaine.

Selon un mode de réalisation, ladite composition utilisée comprend en outre au moins un autre agent dépigmentant, éclaircissant, blanchissant et/ou d'élimination des taches pigmentaires cosmétiquement acceptable.

Selon un mode de réalisation, ledit autre agent dépigmentant, éclaircissant, blanchissant et/ou d'élimination des taches pigmentaires cosmétiquement acceptable est choisi parmi l'acide azélaïque, le péroxyde de benzoyle, le peroxyde d'hydrogène, le méquinol, le trioxopimélate d'éthyle, les analogues de la vitamine A, tels que le béta carotène, la trétinoïne ou le rétinaldéhyde et leurs dérivés, l'acide éthylène diaminetétracétique, le 4-isopropylcatéchol, la diacé-tyl-boldine, les alphahydroxyacides tels que l'acide glycolique, l'acide malique, l'acide citrique, l'acide tartrique, l'acide mandélique ou l'acide lactique, la lutéoline, la vitamine C et ses dérivés tel que l'ascorbyl glucoside, le magnésium ascorbyle phosphate, l'ascorbyl tetraisopalmitate ou l'acide salicylique, l'arbu-tine, l'acide kojique, l'ubiquinone, la vitamine B3 et ses dérivés, le parace-tamol, l'acide linoléique et les huiles végétales en contenant, l'acide ellagique, le mercaptodextran, de la glabridine, de l'hispaglabridine, de la gallotannine, de l'isoliquiritine, de la mélanyde, du N-undecylenoyl-phenylalanine, de la vi-niférine, de l'acide jasmonique et ses dérivés, de l'aloésine, de l'acide alpha lipoïque, les acides dioïques, du glutathion et ses dérivés, les extraits de plantes à flavonoïdes, tel que les extraits d'artichaut, d'achillée millefeuille, de matricaire, de scutellaire, de mandarinier Satsuma, les extraits de la famille des Astéracées, un extrait de busserole, un extrait d'airelle rouge, un extrait de réglisse, un extrait de mûrier, un extrait d'arbousier, un extrait de callune vulgaire, un extrait de pin sylvestre, un extrait de pâquerette, un extrait de vigne, un extrait de grande pimprenelle, un extrait de camomille, un extrait de melon, un extrait de vergerette du Canada, un extrait de piloselle, de l'acide trameti-nique, du sulphoraphane, de l'extrait de cresson et/ou des extraits de saxifrage.

De manière encore plus préférée, ledit au moins un autre agent dépigmentant, éclaircissant, blanchissant et/ou d'élimination des taches pigmentaires cosmétiquement acceptable est choisi parmi la vitamine C, les dérivés de vitamine C, l'arbutine, l'hydroquinone, ou l'acide kojique.

Selon un mode de réalisation, ladite composition utilisée comprend en outre au moins un agent cosmétiquement acceptable choisi parmi les agents kératolytiques, les agents desquamant, les agents apaisants, les filtres solaires, les actifs antirides, les agents hydratants, les agents anti-âges, et/ou les agents tensioactifs.

De manière encore plus préférée, ledit filtre solaire est choisi parmi l'homo-salate, l'oxybenzone, l' ensulizole, l' ecamsul, l' avobenzone, l'acide benzy-lidene camphor sulfonic, octocrylène, le polyacrylamidomethyl benzylidène camphor, l' octinoxate, le peg 25-paba, l'isomamyl p- methoxycinnamate, l' oc-tyl triazone, le drometrizole trisiloxane, diethylhexyl butamido triazone, 4-methylbenzylidene camphor, le 3-benzylidène camphor, l' octisalate, le padi-mate o, benzophenone-4, le methylène bis- benzotriazolyl tetra- methylbu-tylphenol, disodium phenyl dibenzimidazole tetrasulfonate, le bis-ethylhexy-loxyphenol methoxyphenyl triazine, le polysilicone-15, le dioxyde de titane, talc, l'oxyde de zinc, le kaolin, le butyl méthoxydibenzoylméthane, l'octyl-mé-thoxycinnamate, le salicylate d'octyle et/ou le diethylamino hydroxybenzoyl hexyl benzoate.

De manière préférée, la composition cosmétique utilisée selon l'invention est appliquée une à deux fois par jour, de préférence 2 fois par jour, pendant au moins 7 jours, de préférence au moins 15 jours, de manière encore préférée au moins un mois, de manière particulièrement préférée au moins 2 mois, et de manière plus particulièrement préférée au moins 3 mois. De manière tout particulièrement préférée, la composition cosmétique est appliquée selon l'invention 2 fois par jour pendant au moins 3 mois.

Selon un deuxième aspect, l'invention concerne l'utilisation dermatologique non-thérapeutique d'une composition comprenant au moins un acide gras cyclopropénique pour inhiber l'activité de la stéaroyl-CoA désaturase.

Selon l'invention, ladite composition est utilisée pour le traitement non-thérapeutique des hypermélanoses.

De manière préférée, lesdites hypermélanoses sont liées aux mélasmas, aux tâches actiniques, aux lentigos tels que les lentigos solaires et/ou liés au vieillissement, aux pigmentations post-inflammatoires, aux cicatrices pigmentées telles que les cicatrices liées à l'acné, aux lichens pigmentogènes, aux lichens amyloïdes, aux dermatoses cendrées, et/ou aux pigmentations secondaires à la prise de médicaments.

Selon un mode de réalisation, ladite composition pour utilisation dermatologique comprend en outre au moins un autre composé pharmaceutiquement acceptable permettant le traitement des hypermélanoses.

De manière préférée selon l'invention, ladite composition pour utilisation comprend en outre au moins un autre composé pharmaceutiquement acceptable permettant le traitement des hypermélanoses choisi parmi l'hydroquinone et ses dérivés tel que le méquinol, le monométhyl-éther d'hydroquinone et/ou le monobenzylhydroquinone, les corticostéroïdes, les catéchols tels que le 4-isopropyl catéchol et/ou le tertiobutyl catéchol, les composés soufrés tels que la mercaptoéthylamine et/ou le chlorure de béta-mercapto-éthylamine, les dermocorticoïdes tels que la triamcinolone et la bétaméthasone, le 4-S-Cystéamynilphénol, le N-2,4- acétoxyphénylthioéthyl acétamide, l'acide azélaïque, le péroxyde de benzoyle, le peroxyde d'hydrogène, le trioxopimélate d'éthyle, les analogues de la vitamine A tels que le béta carotène, la trétinoïne, ou le rétinaldéhyde et leurs dérivés, l'acide éthylène diaminetétracétique, la diacétyl-boldine, les alphahydroxyacides tels que l'acide glycolique, l'acide malique, l'acide citrique, l'acide tartrique, l'acide mandélique et/ou l'acide lactique, la lutéoline, la vitamine C et ses dérivés tel que l'ascorbyl glucoside, l'ascorbyl tetraisopalmitate, le magnésium ascorbyle phosphate ou l'acide salicylique, l'ar-butine, l'acide kojique, l'ubiquinone, la vitamine B3 et ses dérivés, le paracétamol, l'acide linoléique et les huiles végétales en contenant, l'acide ellagique, le mercaptodextran, le résorcinol et ses dérivés tels que le 4-n-butylrésorcinol,, de la glabridine, de l'hispaglabridine, de la gallotannine, de l'isoliquiritine, de la mélanyde, du N-undecylenoyl-phenylalanine, de la viniférine, de l'acide jasmonique et ses dérivés, de l'aloésine, de l'acide alpha lipoïque, les acides dioïques, du glutathion et ses dérivés, les extraits de plantes à flavonoïdes, tel que les extraits d'artichaut, d'achillée millefeuille, de matricaire, de scutellaire, de mandarinier Satsuma, les extraits de la famille des Astéracées, un extrait de busserole, un extrait d'airelle rouge, un extrait de réglisse, un extrait de mûrier, un extrait d'arbousier, un extrait de callune vulgaire, un extrait de pin sylvestre, un extrait de pâquerette, un extrait de vigne, un extrait de grande pimprenelle, un extrait de camomille, un extrait de melon, un extrait de vergerette du Canada, un extrait de piloselle, et/ou des extraits de saxifrage.

L'hydroquinone est un hydroxyphénol, naturellement présent dans les plantes et les aliments comme le café, les canneberges ou les myrtilles. En raison de son analogie structurelle avec la tyrosine, l'hydroquinone agit comme un substrat alternatif pour la tyrosinase, la réaction générant des ROS, qui sont responsables de son effet éclaircissant. Le méquinol est un de ses dérivés, ainsi que le monométhyl-éther d'hydroquinone ou le monobenzylhydroquinone.

Les corticostéroides sont des hormones stéroïdiennes capables d'inhiber le précurseur alpha-MSH, entraînant une diminution de l'activation de MC1R, résultant en la diminution de la formation de pigments. Des exemples de corticostéroides peuvent être notamment le clobetasolpropionate, l'hydrocortisone acetate, le Bétaméthasone valérate, le Fluocinolone acetonide, la dexametha-sone, la prednisone.

Les dermocorticoïdes agissent en diminuant le nombre de mélanocytes fonctionnels et donc leur activité. Il semblerait aussi que leur action se situe au niveau du passage des granules pigmentaires du mélanocyte aux kératinocytes, par blocage du transfert des mélanosomes.

Les catéchols tels que le 4-isopropyl catéchol et le tertiobutyl catéchol agiraient d'une part en supprimant l'oxydation des intermédiaires de l'eumélanogenèse, d'autre part en activant les enzymes intervenant dans le métabolisme du glutathion pour aboutir à la pheomélanogénèse.

Le 4-S-Cystéamynilphénol et le N-2,4- acétoxyphénylthioéthyl acétamide sont des dérivés soufrés développés afin d'améliorer la mélanocytotoxicité sélective, grâce à la production de radicaux quinone/semi-quinone après interaction avec la tyrosinase. Ces radicaux entrainent des réactions de peroxydation lipidique et la destruction des mélanocytes.

Selon un autre aspect, la description concerne un procédé cosmétique de dépigmentation, d'éclaircissement et/ou de blanchiment d'une zone de la peau, des muqueuses, des poils ou des cheveux humains, et/ou d'élimination des taches pigmentaires de la peau humaine, consistant à appliquer sur ladite zone de la peau, les poils ou les cheveux une composition comprenant au moins un acide gras cyclopropénique pour inhiber l'activité de la stéaroyl-CoA désaturase.

De manière préférée, le procédé cosmétique utilisé est renouvelé une à deux fois par jour, de préférence 2 fois par jour, pendant au moins 7 jours, de préférence au moins 15 jours, de manière encore préférée au moins un mois, de manière particulièrement préférée au moins 2 mois, et de manière plus particulièrement préférée au moins 3 mois. De manière tout particulièrement préférée, la composition cosmétique est appliquée selon l'invention 2 fois par jour pendant au moins 3 mois.

Selon un autre aspect, la description concerne une composition comprenant au moins un acide gras cyclopropénique pour inhiber l'activité de la stéaroyl-CoA désaturase.

Selon un mode de réalisation, ladite composition comprend en outre au moins un autre agent dépigmentant, éclaircissant, blanchissant et/ou d'élimination des taches pigmentaires cosmétiquement acceptable.

Selon un autre aspect, la description concerne une méthode thérapeutique dermatologique comprenant l'application topique d'une composition comprenant au moins un acide gras cyclopropénique pour inhiber l'activité de la stéaroyl-CoA désaturase.

Selon un mode de réalisation, ladite méthode thérapeutique est dans le traitement des hypermélanoses.

De manière préférée, lesdites hypermélanoses sont liées aux mélasmas, aux tâches actiniques, aux lentigos tels que les lentigos solaires et/ou liés au vieillissement, aux pigmentations post-inflammatoires, aux cicatrices pigmentées telles que les cicatrices liées à l'acné, aux lichens pigmentogènes, aux lichens amyloïdes, aux dermatoses cendrées, et/ou aux pigmentations secondaires à la prise de médicaments.

Dans tous les aspects de l'invention, selon un mode de réalisation, ledit au moins un acide gras cyclopropénique est choisi parmi l'acide sterculique et/ou l'acide malvalique et/ou l'acide sterculynique et/ou l'acide hydroxy-2 sterculique, de préférence l'acide sterculique et/ou l'acide malvalique.

Dans tous les aspects de l'invention, de manière préférée, ledit au moins un acide gras cyclopropénique est choisi parmi l'acide sterculique et/ou l'acide malvalique.

Selon un mode de réalisation, lesdites compositions utilisées dans les aspects selon l'invention comprennent un extrait de plantes du genre Gnetum comprenant de l'acide sterculique et/ou de l'acide malvalique.

De manière préférée, ladite plante du genre Gnetum est Gnetum parvifolium ou Gnetum Gnemon.

De manière préférée, lesdits extraits proviennent de la graine, de la feuille, de la fleur, de la pulpe de graine, ou du fruit de ces plantes.

Dans tous les aspects de l'invention, selon un mode de réalisation, l'application est réalisée topiquement sur la peau et/ou sur les muqueuses et/ou sur les poils et/ou sur les cheveux humains.

De manière préférée selon l'invention, lesdites compositions selon l'invention sont formulées sous forme de crème, d'un gel, d'une pommade, d'un onguent, d'un masque, d'un sérum, d'un lait, d'une lotion, d'une pâte, d'une mousse, d'un aérosol, d'un stick, d'un shampooings, d'un d'après-shampooings, de patchs, d'une solution aqueuse hydroalcoolique ou huileuse, d'une émulsion huile-dans-eau ou eau-dans-huile ou multiple, d'un gel aqueux ou huileux, d'un produit anhydre liquide, pâteux ou solide, et/ou d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

La composition utilisée selon de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

La composition utilisée selon l'invention peut être appliquée sur la peau d'un individu.

De manière préférée, la composition est impliquée sur la peau du visage, des bras, du ventre, du dos, des jambes ou des épaules. De manière encore plus préférée selon l'invention, les compositions sont utilisées sur la peau du visage

Les compositions selon l'invention sont appliquées topiquement en quantité suffisante chez l'homme, c'est-à-dire en quantité correspondante aux doses d'application habituelles pour le type de composition considéré (gel, crème, lotion, etc.).

De manière préférée selon l'invention, l'acide gras cyclopropénique est présent en quantité cosmétiquement efficace dans la composition.

De manière préférée selon la description, l'acide gras cyclopropénique est présent en quantité pharmaceutiquement efficace dans la composition.

Selon un mode de réalisation, l'acide gras cyclopropénique est présent dans les compositions et utilisations selon l'invention à une concentration comprise entre 0,01% et 2%, de préférence entre 0,01 et 0,1%, de manière encore plus préférée entre 0,01% et 0,5%, de manière encore plus préférée entre 0,10% et 0,30%, en poids de la composition.

Selon un mode de réalisation, l'acide gras cyclopropénique est présent dans les compositions et utilisations selon l'invention à une concentration de 0,01%, 0,05%, 0,1%, 0,15%, 0,2%, 0,25%, 0,3%, 0,35%, 0,4%, 0,45%, 0,5%, 0,55%, 0,60%, 0,65%, 0,70%, 0,75%, 0,80%, 0,85%, 0,90%, 0,95%, 1%, 1,05%, 1,1%, 1,15%, 1,20%, 1,25%, 1,30%, 1,35%, 1,40%, 1,45%, 1,50%, 1,55%, 1,60%, 1,65%, 1,70%,1,75%, 1,80%, 1,85%, 1,90%, 1,95% ou 2% en poids de la composition.

## Figures

[Fig 1] est un graphique présentant l'effet de l'acide sterculique sur la viabilité. Des concentrations croissantes d'acide sterculique ont été testées sur la viabilité des cellules B16 en utilisant le test de réduction WST-8. Jusqu'à 111 μM, l'acide sterculique n'a pas eu d'effet sur la viabilité.

[Fig 2] est un graphique présentant l'effet de l'acide sterculique sur la mélanogénèse. L'acide sterculique (AcSter) à 100μM et 200 μM induit une inhibition marquée de la teneur en mélanine dans les cellules B16. A 200 μM, l'acide sterculique est aussi efficace que l'acide kojique à 3mM. L'acide sterculique à 200μM n'affecte pas la teneur en protéines de manière significative (Ctl= 7.9±0.1 vs AcSter 7.2 ±0;3 ; p=0.18) ; ce qui indique que l'acide sterculique à cette concentration n'inhibe pas la viabilité des cellules.

**Définitions**

Les « acide gras cyclopéniques » sont des acides gras comprenant un cycle cyclopropène. Les exemples les plus connus sont l'acide malvalique et l'acide sterculique. En général, les deux acides gras sont présents ensemble à des concentrations qui varient jusqu'à 60 % dans les huiles de graines, selon les espèces, et ils sont généralement accompagnés de petites quantités d'analogues cyclopropanoïdes, à savoir les acides dihydrosterculique et dihydromalvalique. Deux autres acides gras cyclopropénoïdes ont été caractérisés dans les huiles de graines, à savoir le 9,10-méthylène-octadec-9-en-17-ynoate ou "acide sterculynique" et l'acide 2-hydroxysterculique, ce dernier étant un intermédiaire possible dans la bioconversion de l'acide sterculique en acide malvalique par un mécanisme d'alpha-oxydation.

L'acide sterculique ou 8-(2-octyl-cyclopropen-1-yl)-octanoic acid (9,10-methy-lene-octadec-9-enoate a pour structure chimique :

[Chem 1]

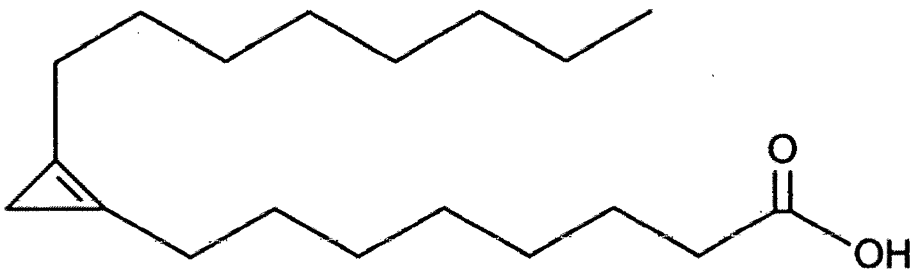

L'acide malvalique ou 7-(2-octyl-cyclopropen-1-yl)-heptanoic acid (9,10-methylene-heptadec-9-enoate a pour structure chimique :

[Chem 2]

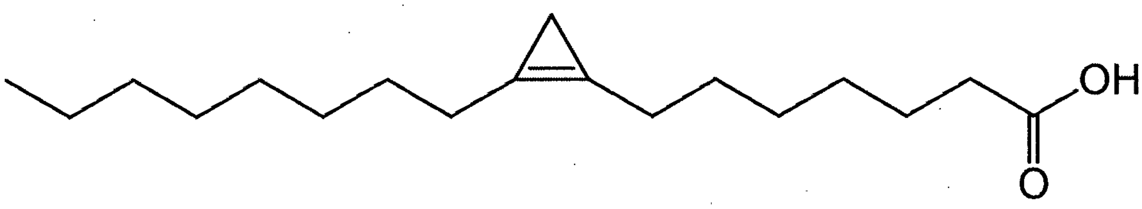

L'acide sterculynique ou méthylène-8,9 octadecène-8cyne-17oïque a pour formule chimique :

[Chem 3]

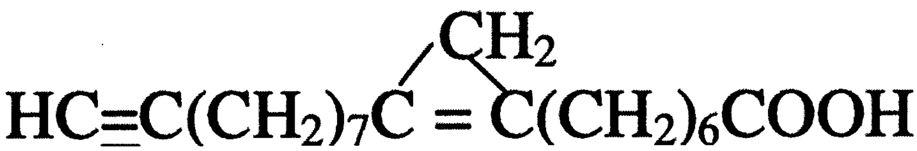

L'acide hydroxy-2 sterculique ou hydroxy-2 méthylène-9,10 octadecène-9 oïque a pour formule chimique :

[Chem 4]

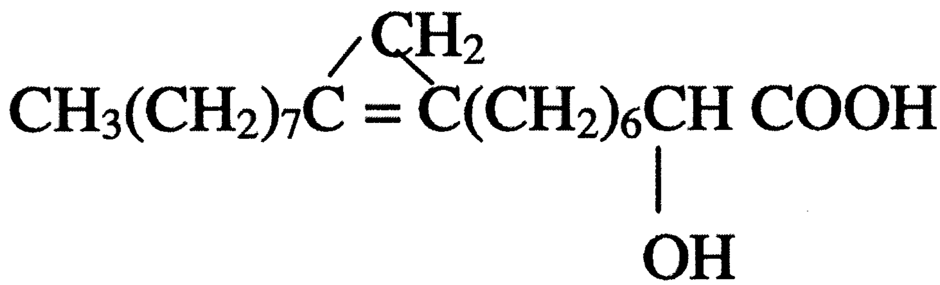

La « stéaroyl-CoA désaturase », « SCD » ou autrement dénommé « FADS5 », « MSTP008 », « SCD1 », « SCDOS », « hSCD1 » est un gène codant pour un enzyme qui introduit une double liaison en position 9 des acides gras saturés, acide palmitique et l'acide stéarique. L'inhibition de l'activité enzymatique de SCD entraine une inhibition de la synthèse de mélanine.

Par « inhiber l'activité » est entendu selon l'invention que l'action de la molécule permet d'empêcher l'action de l'enzyme codée par le gène SCD, à savoir d'introduire une double liaison en position 9 des acides gras saturés.

Par « agent dépigmentant, éclaircissant, blanchissant et/ou d'élimination des taches pigmentaires » est entendu selon l'invention un agent agissant classique-ment sur l'activité des mélanocytes épidermiques où se déroule la mélanogénèse et/ou sur la synthèse et/ou l'activité de la tyrosinase, et/ou interférant avec une des étapes de la biosynthèse de la mélanine ou encore inhibant une des enzymes impliquées dans la mélanogénèse ou agissant comme inhibiteur compétitif d'un des composés de la chaîne de synthèse de la mélanine, entraînant l'inhibition de la synthèse de mélanine, la décoloration des pigmentations cutanées, la couverture des taches, l'éclaircissement du teint mat ou foncé, et/ou l'éclaircissement, le blanchiment, ou la décoloration des muqueuses, des poils ou des cheveux humains. En particulier selon l'invention, les « acide gras cyclopéniques inhibent l'activité de la stéaroyl-CoA désaturase entrainant l'inhibition de la synthèse de la mélanine.

Par élimination des tâches pigmentaires est entendu selon l'invention que la visibilité des tâches pigmentaires est réduite, potentiellement jusqu'à ce qu'elles soient complètement invisibles.

Les agents kératolytique et/ou desquamants aident à éliminer les cellules mortes de la couche cornée de l'épiderme. Il agit par simple contact avec la peau, en interagissant avec la kératine. Des exemples de ces agents sont l'acide salicylique et ses dérivés, l'alpha-hydroxyacides, l'acide ascorbique et ses dérivés et l'urée.

Les agents apaisants ou agents anti-inflammatoires aident à diminuer l'inconfort de la peau et/ou du cuir chevelu. On peut notamment citer l'allantoïne, l'azulène, le bisbolol.

Par filtre solaire ou filtre UV est entendu un agent qui permet de protéger la peau des UVA et/ou des UVB. Elle constitue à ce titre un moyen de photopro-tection externe passive, fonctionnant comme un filtre ultraviolet. Il existe deux types de filtres UV, présent ensemble ou séparément dans les produits commercialisés, à savoir les filtres minéraux comme le talc, l'oxyde de zinc, le dioxyde de titane ou le kaolin, se présentant sous forme de poudres microscopiques qui agissent en réfléchissant les rayons UV, et les filtres chimiques, qui sont des composés chimiques organiques formant un mélange de plusieurs chromophores qui absorbent la lumière ultraviolette (comme l'oxybenzone, le butyl méthoxydibenzoylméthane, l'octyl-méthoxycinnamate, le salicylate d'octyle). Chacun des chromophores possède une bande d'absorption d'UV spécifique (d'où les filtres à bande étroite ou à large bande).

Par agent tensioactif est entendu un agent permettant de solubiliser deux phases non miscibles.

Par hypermélanose est entendu selon l'invention l'accentuation anormale de la pigmentation de la peau, localisée ou généralisée, par augmentation de la quantité de mélanine qu'elle contient. L'hypermélanose est liée à un dépôt en excès de mélanine dans l'épiderme et/ou le derme, et ses causes peuvent diverger.

Ces hypermélanoses peuvent être :

- D'origines génétiques, soit circonscrites dans le cas des taches de café au lait, des éphélides, des lentigos, des lentigos séniles ou taches de sénescences, des taches mongoloïdes, des naevus d'Ota et d'Ito, ou des Naevus de Becker, soit diffuses dans le cas des phacomatoses pigmento-vasculaires et des mélanoses neurocutanée ;
- D'origines endocriniennes, dans le cas de la Maladie d'Addison, des mélasmas, du syndrome de Cushing, du syndrome de Nelson, des phéochromocytomes ;
- D'origines carentielles dans le cas des pellagre et syndromes pellagroïdes, des carences en folates et/ou en vitamine B12, des carences en vitamine C, de l'avitaminose A, des Kwashiorkor, ou des syndromes de malabsorption ;
- D'origines infectieuses, bactériennes dans le cas de l'endocardite ou de la tuberculose, virales dans le cas du VIH, ou parasitaire dans le cas de la leishmaniose viscérale, du paludisme, de la bilharziose ou des anti-paludéens de synthèse ;
- D'origines toxi-médicamenteuses et chimiques, dans le cas des Minocyclines, des Phénothiazines, des Poïkilodermie de Civatte, et/ou des Ochronoses ;

- Liées à des agents physiques, telle la radiothérapie ou des traumatismes répétés ;
- D'origines tumorales ou hémopathiques, dans le cas des mastocytoses, de l'acanthosis nigricans, des Syndromes de Sézary, des mélanomes, de la Maladie de Hodgkin et/ou des Paraprotéinémies ;
- D'origine neurologique, dans le cas Maladie de Parkinson post-encéphalitique et/ou de la Maladie de Schilder ;
- Liées à des maladies systémiques, dans le cas de la Polyarthrite rhumatoïde, de la Maladie de Still et/ou du Lupus érythémateux ;
- Post-inflammatoires, dans le cas des Infections, Impétigos, Brulures, par exemples liées aux UV, cryothérapies, Tréponématoses endémiques, et/ou Phytophotodermatoses, réactions cutanées liées à l'exposition solaire.

De manière préférée selon l'invention, lesdites hypermélanoses sont liées aux mélasmas, aux tâches actiniques, aux lentigos tels que les lentigos solaires et/ou liés au vieillissement, aux pigmentations post-inflammatoires, aux cicatrices pigmentées telles que les cicatrices liées à l'acné, aux lichens pigmentogènes, aux lichens amyloïdes, aux dermatoses cendrées, et/ou aux pigmentations secondaires à la prise de médicaments.

Le mélasma ou chloasma est défini comme une hypermélanose acquise du visage qui peut s'observer dans toutes les ethnies, et dans les deux sexes. Il apparaît cependant plus fréquemment chez les femmes utilisant une contraception orale ou pendant la grossesse (masque de grossesse). Il apparaît ainsi chez les femmes qui ont un taux d'hormones féminines important et dont la peau est exposée au soleil. Il prend la forme de plaques pigmentées de couleur marron, souvent symétriques, de forme plus ou moins régulière.

Les tâches actiniques, autrement dénommées kératoses solaires ou kératoses actiniques, sont des dommages cutanés résultant d'expositions trop prononcées aux rayons UV du soleil ou artificiels. Cette pathologie se caractérise par le développement de tâches de couleur rosâtre, rouge ou brune et pouvant être de tailles variables, résultant de la disparition de fragments de peau.

Les lentigos sont des tâches hyperpigmentées pouvant siéger sur la peau, les ongles ou les muqueuses. On parle de lentigo solaire pour les zones les plus fréquemment photo-exposées (visage, mains, décolleté), et de lentigo sénile, lié au vieillissement cutané, qui sont des taches pigmentaires assez larges, qui apparaissent chez le sujet âgé au niveau des mains, du visage et des bras. Ces taches sont dues à une concentration importante de mélanine dans les kératinocytes situés à la surface de la peau.

Les pigmentations post-inflammatoires résultent d'une surproduction de mélanine suite à une inflammation de la peau. Elles se caractérisent par une accumulation de pigments mélaniques au niveau de la blessures cutanées, qui peut être causée par tout type de blessure cutanée comme l'acné, une dermatite ou une blessure traumatique, et peut apparaitre sur les couches cutanées et / ou épidermiques.

Les cicatrices pigmentées résultent d'une mauvaise cicatrisation pouvant par exemple être liée à des boutons d'acné, et forment ainsi une accumulation de mélanine entraînant des taches brunes sur la peau.

Le lichen plan est une maladie inflammatoire de la peau et/ou de la muqueuse de la bouche. Les lichens pigmentogènes ou « lichen plan pigmentosa » sont des variantes des lichens plans caractérisés par une hyperpigmentation macu-leuse dans les zones photo-exposées ou dans les plis de flexions, et principalement localisées au visage et sur les membres supérieurs.

Le lichen amyloïde est une forme chronique et rare d'amylose cutanée caractérisée par l'accumulation de dépôts amyloïdes dans le derme. Cette pathologie se manifeste par le développement de papules prurigineuses hyperkératotiques souvent pigmentées sur le tronc et les extrémités, surtout au niveau des tibias.

Les dermatoses cendrées sont des dermatoses caractérisées par des tâches de couleur bleuâtre à gris centre, ainsi que des macules de diverses teintes de gris présentant un bord érythémateux légèrement surélevé se développant essentiellement sur le visage, le tronc et les membres supérieurs.

Par « pigmentation secondaire à la prise de médicaments » est entendu selon l'invention des taches pigmentaires qui apparaissent à la suite de la prise de médicament et pouvant résulter notamment d'une réaction de photosensibilisation. Les minocyclines ou les phénothiazines sont des exemples de médicaments entraînant ce type de pigmentation.

Par « non-thérapeutique » est entendu au sens de l'invention, une application cosmétique n'ayant pas vocation à traiter un patient. De fait, l'utilisation cosmétique selon l'invention n'agit pas comme un médicament pour le traitement de maladies, mais seulement pour atténuer les signes visuels de pigmentation sur la peau.

Par « application topique », on entend au sens de la présente invention une application sur la peau (dont le cuir chevelu), et les muqueuses.

Par « cosmétiquement acceptable », on entend au sens de la présente invention ce qui est utile dans la préparation d'une composition cosmétique, qui est généralement sûr, non toxique et ni biologiquement ni autrement non souhaitable et qui est acceptable pour une utilisation cosmétique, notamment par application topique au niveau de la peau.

Par « pharmaceutiquement acceptable », on entend ce qui est utile dans la préparation d'une composition

pharmaceutique, qui est généralement sûr, non toxique et ni biologiquement ni autrement non souhaitable pour un être humain et qui est acceptable pour une utilisation pharmaceutique dermatologique, notamment par application topique au niveau de la peau.

Par « quantité cosmétiquement » efficace est entendu une quantité permettant d'obtenir les effets revendiqués selon l'invention.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les exemples ci-après sont présentés à titre illustratif et non limitatif du domaine de l'invention.

Exemples

Exemple 1 : Évaluation de l'effet de l'acide sterculique sur la synthèse de la mélanine dans les cellules de mélanome murin B16 stimulées par le NDP-MSH

*Introduction*

Dans la présente étude, l'effet blanchissant potentiel de l'acide sterculique a été évalué sur la synthèse de mélanine dans les cellules de mélanome murin B16 stimulées par un analogue stable de l'$\alpha$-MSH, le NDP-MSH ([Nle4, D-Phe7]-$\alpha$-Melanocyte Stimulating Hormone).

Le modèle de mélanocyte B16 constitue un modèle approprié pour la recherche d'effets inhibiteurs sur la production de mélanine car il est très réactif (par exemple à une stimulation NDP-MSH), ce qui entraîne une forte modulation de la production de mélanine entre les conditions basales et les conditions stimulées. Cette forte production de mélanine dans des conditions stimulées permet d'identifier les composés présentant des propriétés blanchissantes.

Avant cet essai, un essai de cytotoxicité a été réalisé à l'aide d'un essai standard de réduction WST-8, afin de déterminer les concentrations à tester dans cette étude.

*Matériels et méthodes*

*Modèle biologique :*

Type cellulaire : Lignée cellulaire de mélanome murin B16 (= mélanocytes B16)
Conditions de culture : 37°C, 5% CO2
Milieu de culture : DMEM sans rouge de phénol optimisé pour l'essai, complété par du sérum de veau fœtal à 10%.

*Composé testé : acide sterculique*
50, 100 et 200 $\mu$M (dans 0.066% ethanol)
*Essai préliminaire de cytotoxicité*

- Type de cellule : Cellules de mélanome murin B16 en milieu de culture
- Durée d'incubation : 72 heures
- Paramètres d'évaluation : Essai de réduction WST-8 et observations morphologiques à l'aide d'un microscope. Après traitement, les cellules sont incubées avec du WST-8 (sel de tétrazolium très soluble dans l'eau) réduit en un produit soluble dans l'eau de couleur orange (formazan) par la succinate déshydrogé-nase (enzyme mitochondriale). Cette transformation est proportionnelle au nombre de cellules vivantes et à leur activité métabolique. La densité optique (DO) des extraits à 450 nm a été enregistrée avec un spectromètre (VERSAmax, Molecular Devices).

*Culture et traitement*

Des cellules de mélanome B16 ont été ensemencées dans une plaque à 96 puits et incubées dans un milieu de culture pendant 24 heures.

Le milieu a ensuite été remplacé par du milieu de culture, en présence de NDP-MSH à 0,1 $\mu$M, contenant ou non :
- le solvant témoin (éthanol testé à 0,066 %),

- le composé d'essai acide sterculique testé à 50, 100 ou 200 $\mu$M,

- l'acide kojique de référence testé à 0,5 mM ou 3 mM,
- la combinaison du composé testé acide sterculique (50, 100 ou 200 μM) et de l'acide kojique à 0,5 mM.

Les cellules ont été incubées pendant 72 heures. En parallèle, une condition de contrôle non stimulée a été réalisée.
Toutes les conditions expérimentales ont été réalisées en n=3.
Le milieu contenant ou non le composé testé, l'acide kojique ou leur combinaison a été ajouté aux puits non ensemencés afin de quantifier l'interférence potentielle des composés. Ces contrôles d'interférence ont été effectués dans n=1.

*Évaluation du contenu de l'échantillon*

À la fin du temps d'incubation, l'absorbance à 405 nm a été mesurée et la teneur totale en mélanine (extracellulaire et intracellulaire) a été calculée pour chaque échantillon à l'aide d'une courbe standard de mélanine allant de 0,78 à 100 μg/ml.
Les valeurs de fond (interférences potentielles), mesurées dans les puits sans cellules, ont été soustraites aux valeurs calculées afin de considérer l'effet lié à la production de mélanine uniquement.
Les résultats ont été exprimés en pourcentage de mélanine par rapport au contrôle stimulé.

*Quantité de protéines totales*

La quantité de protéines totales contenues dans les cellules et les surnageants de chaque réplicat a été mesurée à l'aide du kit DC Protein Assay contre une gamme de BSA avec un lecteur de microplaques à 750 nm (VERSAmax, Molecular Devices).
Kit : DC Protein Assay de BIO-RAD ref 500-0116.
Limite de détection basse : 0,2 mg/ml Limite de détection haute : 2 mg/ml
Pour chaque condition de test, la quantité de mélanine a été normalisée par rapport à la quantité de protéines totales.

*Gestion des données*

Les données brutes ont été analysées à l'aide du logiciel Microsoft Excel®.
Les comparaisons entre groupes ont été effectuées à l'aide d'un test t de Student. L'analyse statistique peut être interprétée si n≥5, mais pour n<5, les valeurs statistiques sont données à titre indicatif.

Formules utilisées dans ce rapport :

Erreur standard de la moyenne : $\text{sem} = \text{Sd}/\sqrt{n}$
L'erreur standard de la moyenne (sem) est une mesure de la distance probable entre la moyenne de l'échantillon et la moyenne réelle de la population. Elle est calculée en divisant l'écart-type par la racine carrée de la taille de l'échantillon.

$$\text{Pourcentage de viabilité : viabilité (\%)} = (\text{OD échantillon} / \text{OD contrôle}) \times 100$$

Pourcentage Inhibition relative (%) = ( (Moyenne du contrôle stimulé - Valeur) /(Moyenne du contrôle stimulé - Moyenne du contrôle non stimulé) ) × 100

*<u>Résultats</u>*

*Essai préliminaire de cytotoxicité*

[Table 1]

| AcSter, $\mu$M | 0 | 1,5 | 12 | 37 | 111 | 333 | 1000 |
|---|---|---|---|---|---|---|---|
| % de cellules viables | 97,76734 | 133,9982 | 112,1381 | 82,74032 | 112,2056 | 26,41552 | 1,061339 |
| | 104,6777 | 119,6442 | 146,2746 | 102,0043 | 119,9363 | 44,06769 | 1,910951 |
| | 97,55502 | 122,5711 | 110,4588 | 72,79147 | 106,0771 | 52,32917 | 2,113628 |

Les résultats de l'essai de réduction WST-8 et l'observation des couches cellulaires ont permis de déterminer, en accord avec le commanditaire de l'étude, les concentrations à tester (voir ci-dessus) (Figure 1).

*Effet sur la synthèse de la mélanine*

[Table 2]

| | | NDP-MSH | | | | | | |
|---|---|---|---|---|---|---|---|---|
| | CTRL non-stimulé | CTRL | CTRL + etho | AS, 50 $\mu$M | AS, 100 $\mu$M | AS, 200 $\mu$M | KA, 0,5 mM | KA, 3 mM |
| Teneur en mélanine | 8,267716 | 100 | 97,76903 | 103,1496 | 57,21785 | 13,64829 | 31,36483 | 17,4540 |
| | 10,02674 | 100 | 109,2246 | 125,1337 | 56,01605 | 13,90374 | 49,33155 | 20,7219 |
| | 10,09682 | 100 | 99,44675 | 106,9156 | 61,96404 | 21,16183 | 42,04703 | 19,5020 |

Les résultats sont également présentés en Figure 2.

Le traitement des mélanocytes B16 par le NDP-MSH, testé à 0,1 $\mu$M, a induit une forte stimulation de la synthèse de mélanine par rapport à la condition contrôle non stimulée.

L'acide kojique de référence (KA), testé à 3 mM, a fortement inhibé l'effet du NDP-MSH (89 % d'inhibition relative). Ce résultat était attendu et a validé le test. À 0,5 mM, l'acide kojique a induit une inhibition modérée de la synthèse de mélanine dans les cellules B16 stimulées par le NDP-MSH (65 % d'inhibition relative).

Le solvant témoin, l'éthanol, testé à 0,066 %, n'a eu aucun effet sur la synthèse de mélanine dans les cellules B16 stimulées par le NDP-MSH.

L'acide sterculique (AS) n'a eu aucun effet à 50 $\mu$M mais a induit une inhibition forte et dépendante de la concentration de la synthèse de mélanine à 100 $\mu$M et 200 $\mu$M (jusqu'à 93 % d'inhibition relative).

La combinaison de l'acide sterculique avec l'acide kojique à 0,5 mM a induit une inhibition forte et concentration-dépendante de la synthèse de mélanine dans les cellules B16 stimulées par le NDP-MSH (jusqu'à 97 % d'inhibition relative à la concentration la plus élevée). Ces effets étaient plus prononcés avec la combinaison qu'avec les composés testés seuls.

*Conclusion*

En conclusion, l'acide sterculique, seul ou en combinaison avec l'acide kojique, a démontré un effet en tant qu'agent dépigmentant, éclaircissant et/ou blanchissant de la peau, des muqueuses, des poils et/ou des cheveux humains et/ou comme agent permettant d'éliminer les taches pigmentaires de la peau humaine.

## Revendications

1. Utilisation cosmétique non-thérapeutique d'une composition comprenant au moins un acide gras cyclopropénique pour inhiber l'activité de la stéaroyl-CoA désaturase, où ledit acide gras cyclopropénique agit comme agent dépigmentant, éclaircissant et/ou blanchissant de la peau, des muqueuses, des poils et/ou des cheveux humains et/ou comme agent permettant d'éliminer les taches pigmentaires de la peau humaine.

2. Utilisation cosmétique non-thérapeutique selon la revendication précédente, comprenant en outre au moins un autre agent dépigmentant, éclaircissant, blanchissant et/ou d'élimination des taches pigmentaires cosmétiquement

acceptable.

3. Utilisation cosmétique non-thérapeutique selon l'une quelconque des revendications précédentes, comprenant en outre au moins un agent cosmétiquement acceptable choisi parmi les agents kératolytiques, les agents desquamant, les agents apaisants, les filtres solaires, les actifs antirides, les agents hydratants, les agents anti-âges, et/ou les agents tensioactifs.

4. Utilisation cosmétique non-thérapeutique selon l'une quelconque des revendications précédentes, dans laquelle l'acide gras cyclopropénique est présent dans les compositions et utilisation selon l'invention à une concentration comprise entre 0,01% et 2%, de préférence entre 0,01 et 0,1%, de manière encore plus préférée entre 0,01% et 0,5%, de manière encore plus préférée entre 0,10% et 0,30%, en poids de la composition.

5. Utilisation dermatologique non-thérapeutique d'une composition comprenant au moins un acide gras cyclopropénique pour inhiber l'activité de la stéaroyl-CoA désaturase, ladite composition étant utilisée pour le traitement des hypermélanoses.

6. Utilisation dermatologique non-thérapeutique selon la revendication précédente, où lesdites hypermélanoses sont liées aux mélasmas, aux tâches actiniques, aux lentigos tels que les lentigos solaires et/ou liés au vieillissement, aux pigmentations post-inflammatoires, aux cicatrices pigmentées telles que les cicatrices liées à l'acné, aux lichens pigmentogènes, aux lichens amyloïdes, aux dermatoses cendrées, et/ou aux pigmentations secondaires à la prise de médicaments.

7. Utilisation dermatologique non-thérapeutique selon l'une quelconque des revendications 5 et 6, comprenant en outre au moins un autre composé pharmaceutiquement acceptable permettant le traitement des hypermélanoses.

8. Utilisation cosmétique ou dermatologique non-thérapeutique selon l'une quelconque des revendications 1 à 7, où ledit au moins un acide gras cyclopropénique est choisi parmi l'acide sterculique et/ou l'acide malvalique, et/ou l'acide sterculynique et/ou l'acide hydroxy-2 sterculique, de préférence l'acide sterculique et/ou l'acide malvalique.

9. Utilisation cosmétique ou dermatologique non-thérapeutique selon la revendication 8, comprenant un extrait de plantes du genre Gnetum comprenant de l'acide sterculique et/ou de l'acide malvalique.

10. Utilisation cosmétique ou dermatologique non-thérapeutique selon la revendication précédente, où ladite plante du genre Gnetum est Gnetum parvifolium ou Gnetum Gnemon.

11. Utilisation cosmétique ou dermatologique non-thérapeutique selon l'une quelconque des revendications 1 à 10 appliquée topiquement sur la peau et/ou sur les muqueuses et/ou sur les poils et/ou sur les cheveux humains.

## Patentansprüche

1. Nicht-therapeutische kosmetische Verwendung einer Zusammensetzung umfassend mindestens eine Cyclopropenfettsäure zur Hemmung der Aktivität der Stearoyl-CoA-Desaturase, wobei die genannte Cyclopropenfettsäure als depigmentierendes, aufhellendes und/oder bleichendes Mittel für die Haut, die Schleimhäute, die Haare und/oder das Kopfhaar des Menschen und/oder als Mittel zur Beseitigung von Pigmentflecken der menschlichen Haut wirkt.

2. Nicht-therapeutische kosmetische Verwendung nach dem vorstehenden Anspruch, ferner umfassend mindestens ein anderes kosmetisch verträgliches depigmentierendes, aufhellendes, bleichendes und/oder Pigmentflecken beseitigendes Mittel.

3. Nicht-therapeutische kosmetische Verwendung nach einem der vorstehenden Ansprüche, ferner umfassend mindestens ein kosmetisch verträgliches Mittel, ausgewählt aus keratolytischen Mitteln, schuppenlösenden Mitteln, beruhigenden Mitteln, Sonnenschutzmitteln, Anti-Falten-Wirkstoffen, feuchtigkeitsspendenden Mitteln, Anti-Aging-Mitteln und/oder tensidischen Mitteln.

4. Nicht-therapeutische kosmetische Verwendung nach einem der vorstehenden Ansprüche, wobei die Cyclopropenfettsäure in den erfindungsgemäßen Zusammensetzungen und Verwendung in einer Konzentration zwischen 0,01 % und 2 %, vorzugsweise zwischen 0,01 und 0,1 %, noch bevorzugter zwischen 0,01 % und 0,5 %, noch bevorzugter

zwischen 0,10 % und 0,30 %, bezogen auf das Gewicht der Zusammensetzung, vorliegt.

5. Nicht-therapeutische dermatologische Verwendung einer Zusammensetzung umfassend mindestens eine Cyclopropenfettsäure zur Hemmung der Aktivität der Stearoyl-CoA-Desaturase, wobei die Zusammensetzung zur Behandlung von Hypermelanosen verwendet wird.

6. Nicht-therapeutische dermatologische Verwendung nach dem vorstehenden Anspruch, wobei die genannten Hypermelanosen mit Melasmen, aktinischen Flecken, Lentigos wie Solarlentigos und/oder alterungsbedingten Lentigos, postinflammatorischen Pigmentierungen, pigmentierten Narben wie aknebedingten Narben, pigmentogenen Flechten, amyloiden Flechten, aschgrauen Dermatosen und/oder sekundären Pigmentierungen durch Medikamenteneinnahme verbunden sind.

7. Nicht-therapeutische dermatologische Verwendung nach einem der Ansprüche 5 und 6, ferner umfassend mindestens eine weitere pharmazeutisch verträgliche Verbindung, die die Behandlung von Hypermelanosen ermöglicht.

8. Nicht-therapeutische kosmetische oder dermatologische Verwendung nach einem der Ansprüche 1 bis 7, wobei die mindestens eine cyclopropenische Fettsäure ausgewählt ist aus Sterculinsäure und/oder Malvalsäure und/oder Sterculynsäure und/oder 2-Hydroxy-Sterculinsäure, vorzugsweise Sterculinsäure und/oder Malvalsäure.

9. Nicht-therapeutische kosmetische oder dermatologische Verwendung nach Anspruch 8, umfassend einen Pflanzenextrakt der Gattung Gnetum, der Sterculinsäure und/oder Malvalsäure umfasst.

10. Nicht-therapeutische kosmetische oder dermatologische Verwendung nach dem vorstehenden Anspruch, wobei die Pflanze der Gattung Gnetum Gnetum parvifolium oder Gnetum Gnemon ist.

11. Nicht-therapeutische kosmetische oder dermatologische Verwendung nach einem der Ansprüche 1 bis 10, topisch auf die Haut und/oder auf die Schleimhäute und/oder auf die Haare und/oder auf das Kopfhaar des Menschen aufgetragen.

## Claims

1. Non-therapeutic cosmetic use of a composition comprising at least one cyclopropene fatty acid for inhibiting stearoyl-CoA desaturase activity, where said cyclopropene fatty acid acts as a depigmenting, lightening and/or whitening agent for human skin, mucous membranes, body hair and/or hair and/or as an agent for removing pigment spots from human skin.

2. Non-therapeutic cosmetic use according to the preceding claim, further comprising at least one other cosmetically acceptable depigmenting, lightening, whitening and/or pigment spot removal agent.

3. Non-therapeutic cosmetic use according to either of the preceding claims, further comprising at least one cosmetically acceptable agent selected from keratolytic agents, desquamating agents, soothing agents, sunscreens, anti-wrinkle active ingredients, moisturizing agents, anti-aging agents, and/or surfactants.

4. Non-therapeutic cosmetic use according to any of the preceding claims, wherein the cyclopropene fatty acid is present in the compositions and use according to the invention at a concentration of between 0.01% and 2%, preferably between 0.01% and 0.1%, even more preferably between 0.01% and 0.5%, even more preferably between 0.10% and 0.30%, by weight of the composition.

5. Non-therapeutic dermatological use of a composition comprising at least one cyclopropene fatty acid for inhibiting stearoyl-CoA desaturase activity, said composition being used for the treatment of hypermelanoses.

6. Non-therapeutic dermatological use according to the preceding claim, where said hypermelanoses are related to melasmas, actinic spots, lentigines such as solar and/or age-related lentigines, post-inflammatory pigmentations, pigmented scars such as acne-related scars, pigmentogenic lichens, amyloid lichens, ashy dermatoses, and/or pigmentations caused by taking medication.

7. Non-therapeutic dermatological use according to any of claims 5 and 6, further comprising at least one other

pharmaceutically acceptable compound for the treatment of hypermelanoses.

**8.** Non-therapeutic cosmetic or dermatological use according to any of claims 1 to 7, where said at least one cyclopropene fatty acid is selected from sterculic acid and/or malvalic acid and/or sterculynic acid and/or 2-hydroxy sterculic acid, preferably sterculic acid and/or malvalic acid.

**9.** Non-therapeutic cosmetic or dermatological use according to claim 8, comprising an extract of plants of the genus Gnetum comprising sterculic acid and/or malvalic acid.

**10.** Non-therapeutic cosmetic or dermatological use according to the preceding claim, where said plant of the genus Gnetum is Gnetum parvifolium or Gnetum Gnemon.

**11.** Non-therapeutic cosmetic or dermatological use according to any of claims 1 to 10 applied topically to human skin and/or mucous membranes and/or body hair and/or hair.

[Fig 1]

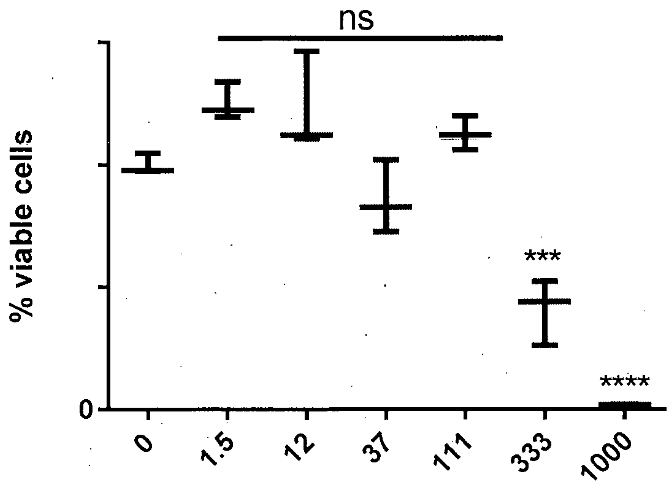
ns
% viable cells
0
***
****
0
1.5
12
37
111
333
1000

[Fig 2]

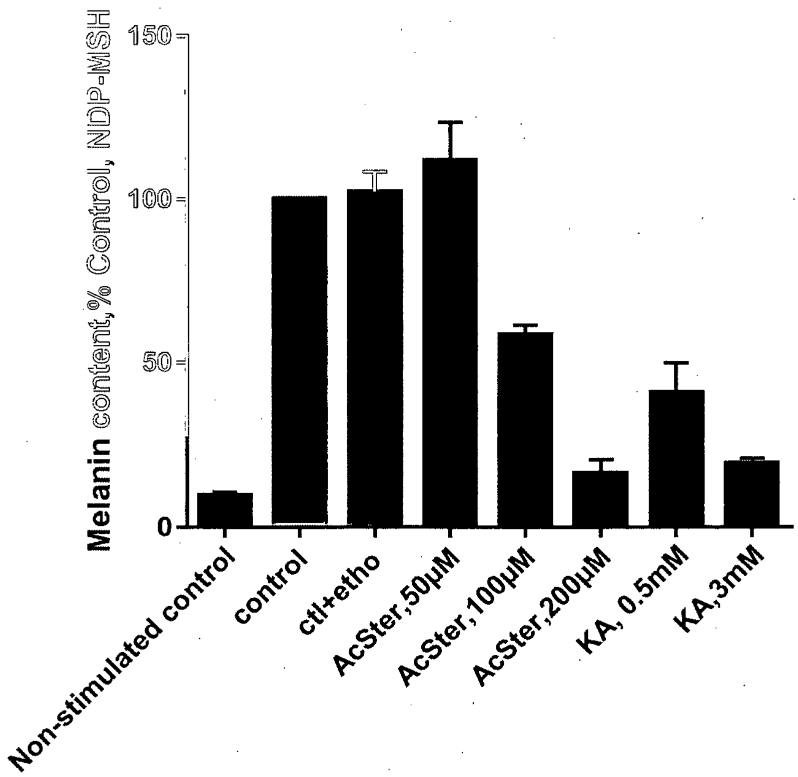
Melanin content,% Control, NDP-MSH
150
100
50
0
Non-stimulated control
control
ctl+etho
AcSter,50μM
AcSter,100μM
AcSter,200μM
KA, 0.5mM
KA,3mM

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2018142033 A


### Littérature non-brevet citée dans la description


- **AYUNINGTYAS et al.** *Pharmacognosy Research*, 2018, 432-436